# EUROPEAN PATENT SPECIFICATION

(11) **EP 1 057 480 B1**
(45) Date of publication and mention of the grant of the patent: **23.06.2004**
(21) Application number: 00304611.7
(22) Date of filing: 30.05.2000
(51) Int. Cl.: A61K 9/70, A61K 31/40, A61P 5/32, A61K 31/439, A61K 31/445

(54) **Transdermal estrogen agonist-antagonist therapy**
Transdermale Östrogen Agonist-Antagonist Therapie
Thérapie transdermique à base d'un agoniste-antagoniste d' oestrogène

(30) Priority: 01.06.1999 US 137164 P
(43) Date of publication of application: 06.12.2000
(73) Proprietor: Pfizer Products Inc., Groton, Connecticut 06340 (US)
(72) Inventor: Da Silva-Jardine, Paul Andrew, Groton, Connecticut 06340 (US)
(74) Representative: Simpson, Alison Elizabeth Fraser

(56) References cited:
- EP-A- 0 842 661
- EP-A- 0 843 999
- US-A- 5 605 700
- US-A- 5 904 930

## Description

### FIELD OF THE INVENTION

This invention relates to transdermal estrogen agonist-antagonist (SERM) therapy for humans in need of such treatment. In particular, this invention relates to a method which involves transdermal delivery of 5-substituted-6-cyclic-5,6,7,8-tetrahydronaphthalene-ol compounds which are estrogen agonists-antagonists effective for treating or preventing conditions related to estrogen imbalance including breast cancer, osteoporosis, obesity, cardiovascular disease, hypercholesterolemia, endometriosis and prostate disease.

### DESCRIPTION OF RELATED ART

Transdermal drug delivery systems are known in the art. Some patents in this area are concerned with the transdermal delivery of hormone formulations for various purposes. Examples of such patents are as follows:

U.S. Pat. No. 4,816,258 issued Mar. 28, 1989 to Nederberge et al discloses a drug formulation of ethinyl estradiol and levo-norgestrel for transdermal administration which comprises a matrix containing the steroids and a skin permeation enhancing amount of glycerol monoleate.

U.S. Pat. No. 5,122,382 issued Jun. 16, 1992 to Gail et al discloses a composition for transdermal administration comprising an estrogen and ST-1435. The formulation delivers certain quantities per day of the steroids and includes a skin permeation enhancer.

U.S. Pat. No. 5,023,084 issued Jun. 11, 1991 and U.S. Pat. No. 4,906,169 issued Mar. 6, 1992, both to Chien et al describe a combination of estrogen and progestin in a contraceptive formulation with a transdermal unit comprising a backing layer containing the hormones and an adhesive layer.

U.S. Pat. Nos. 4,624,665, issued Nov. 25, 1986; 4,687,481 issued Aug. 18, 1987; 4,834,978 issued May 30, 1989; and, 4,810,499 issued Mar. 7, 1989 and 4,927,687 issued May 22, 1990 describe a transdermal delivery system which is said to prevent dose dumping of the drug to be delivered caused by accidental rupture of a retaining member and ensures effective and prolonged delivery of the drug, particularly contraceptive steroids. Suitable steroids include norethindrone, norgestrel, estradiol, levo-norgestrel and mestranol.

U.S. Pat. No. 5,422,119 describes a method of transdermally administering to a female in need of hormone replacement therapy a series of estrogen and progestin in alternate phases.

U.S. Pat. No. 5,552,412 describes the estrogen agonist-compounds which are employed in this invention.

W097/43989 describes hydrogel compositions which are useful for transdermal delivery of drugs.

EP 0 328 806 describes a transdermal therapeutic system without membrane; its matrix consists of a polyacrylate adhesive, a solvent, a penetration enhancer, and estrogen, its derivatives and combinations thereof.

WO 87/07 138 describes an estradiol patch having a backing layer, an active substance-containing matrix, and a pressure-sensitive adhesive covered with a removable protective layer. Matrix and pressure-sensitive adhesive are manufactured in operations involving considerable technological expenditure, i.e., by homogenizing, degassing, coating, drying, and separating. According to an embodiment the backing layer has to be coated with a pressure-sensitive adhesive, requiring an additional operation. The individual parts are joined in a separate step. For this reason, the production of this patch is very expensive and complicated.

EP 0 186 019 describes active substance patches wherein water-swellable polymers are added to a rubber/adhesive-resin-mass and from which estradiol can be released. It turned out, however, that the estradiol release from these active substance patches is absolutely insufficient and fails to meet the therapeutic requirements.

DE-OS 20 06 969 describes a patch or a pressure-sensitive adhesive dressing exhibiting system action, wherein contraceptive substances are incorporated in the adhesive component or in the adhesive film. The adhesive film may be an acrylate.

### SUMMARY OF THE INVENTION

This invention provides for the use of an estrogen agonist-antagonist for the preparation of a medicament for the treatment by transdermal means of a human in need of estrogen agonist-antagonist therapy.

This invention also provides for the use of an estrogen agonist-antagonist for the preparation of a medicament for the treatment by transdermal means of a human in need of estrogen agonist-antagonist therapy wherein said estrogen agonist-antagonist is a compound having the formula wherein G is 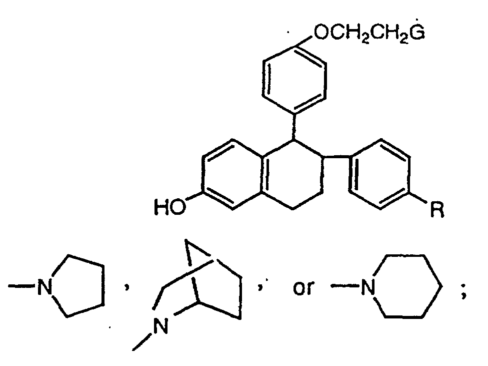 and R is H, OH. F or Cl.

The invention further provides for the use of said estrogen agonist-antagonist of the abovementioned formula for the preparation of a medicament for the treatment by transdermal means of a human in need of estrogen agonist-antagonist therapy wherein said transdermal means is a transdermal patch or a hydrogel composition.

This invention further provides a transdermal patch which provides administration of said estrogen agonist-antagonist to said human over a period of three to fourteen days in substantially equal amounts of 10-500 µg per day.

This invention further provides a transdermal patch comprising a matrix and a pharmaceutically effective amount of (-)-cis-6-phenyl-5-[4-(2-pyrrolidin-1-yl-ethoxy)-phenyl]-5,6,7,8-tetrahydronaphthalene-2-ol or a pharmaceutically acceptable salt thereof.

In a preferred form of the present invention a patch of an adhesive matrix type or a drug reservoir type is prepared containing sufficient estrogen agonist-antagonist to provide a transdermal delivery rate of about 10-500 µg per day.

The selection of estrogen agonists-antagonists most suited for transdermal delivery may be determined by conventional tests used in the art to determine skin permeability. The most common is the In Vitro Skin Protection Chamber using hairless mouse skin or human cadaver skin. The literature contains much information on these tests and the following references are typical:
a) Kao J, Hall J. Skin Absorption and Cutaneous First Pass Metabolism of Topical Steroids: In Vitro Studies with Mouse Skin in Organ Culture. J Pharmacol Exp Ther 241 (2), pp 482-487, 1987;
b) Tojo K, Lee C. A Method for Predicting Steady-state Rate of Skin Penetration In Vivo. J Invest Derm 92(1 ), pp 105-108, 1989;
c) Liu P, Higuchi WI, Song W, Kurihara-Bergstrom T, Good WR. Quantitative Evaluation of Ethanol Effects on Diffusion and Metabolism of β-Estradiol in Hairless Mouse Skin. Pharm. Res 8(7), pp 865-872, 1991; and
d) Roy S, Gutierrez M, Chiang C. Permeation of Norethindrone, Norethindrone Acetate and Norethindrone Diacetate Through Cadaver Skin. Pharm Res Suppl 6, p 1168,1989.

In selecting suitable estrogen agonists-antagonists, skin permeation and hence transdermal delivery rates are important, but also, the stability of the estrogen agonists in the formulation can be a factor. Stability can be assessed or measured in two basic ways: visual inspection and in vitro dissolution.

Visual inspection is generally conducted on both a macroscopic and a microscopic level of crystal formation. As for in vitro dissolution, there are tests for in vitro release rates which are standard in the art. Generally when these criteria are met, the transdermal delivery rates required are readily achieved.

There are a number of transdermal patches available commercially which may be used in the present method. Two such patches are described here for purposes of exemplication only, but others may be employed.

In one known system, a matrix of a solid adhesive of acrylic copolymer base containing hormones in dissolved form is employed. The system involves the use of a flexible backing foil which may comprise for example polyethylene terephthalate. A release liner forms another layer (in contact with the adhesive). A swellable polysaccharide, galactomannan is added to the adhesive matrix to improve the adhesion between the surface of the patch and hydrated skin, over the whole application, and to reduce irritation phenomena caused by occlusion effects. Preferably, a three layer laminate is produced of the backing foil, drug matrix and release liner. Currently, a commercial product of this nature is sold under the trade mark SYSTEN TTS.

Another commercial product is that available under the trade mark ESTRADERM. ESTRADERM™ is an estradiol transdermal system designed to release estradiol transdermal system designed to release estradiol through a rate-limiting- membrane continuously upon application to intact skin. The ESTRADERM™ system comprises five layers: a backing layer, a drug reservoir, a control membrane, an adhesive layer and a protective layer in that order from outside to inside. The qualitative composition of the ESTRADERM drug reservoir is estradiol, alcohol and hydroxypropyl cellulose.

For transdermal administration, as has already been stated, any suitable form of patch may be selected. Another which is known to be useful in the administration of hormone formulations is described in detail in U.S. Pat. No. 4,668,232 issued May 26,1987 to Cordes and Wolff. In this U.S. patent, the system described is a four layer system comprising an impermeable backing or covering layer; a reservoir layer adjacent to to the backing or covering layer; an adhesive layer permeable to the hormones and a protective layer which is removed before application of the patch. As described earlier, a three layer system may be used which comprises a backing layer, a drug matrix adhesive layer and a release liner.

An estrogen agonist-antagonist patch for the present invention may be prepared using an ethanolic gel type of drug reservoir. The weight of hormone, preferably (-)-cis-6-phenyl-5-[4-(2-pyrrolidin-1-yl-ethoxy)-phenyl]-5,6,7,8-tetrahydronaphthalene-2-ol is about 140-7000 µg. The patch may have a surface area of about 18 cm². In appearance it may be ground with the reservoir comprising about 10 cm² of the patch. This generally provides a transdermal delivery rate of about 10-500 µg per day.

### DETAILED DESCRIPTION OF THE INVENTION

In a preferred mode, the present invention relates to an active substance-containing patch for the controlled release of an estrogen agonist amount or pharmaceutically acceptable derivatives alone or combined with other compounds, consisting of a backing layer, an active substance-containing reservoir which is bonded thereto and is produced by using pressure-sensitive adhesive comprising ethylcellulose, esters of non-hydrogenated and/or hydrogenated colophony, and lauric acid.

Ethylcellulose is a cellulose ether produced by reacting ethyl chloride with alkali cellulose. With respect to the structure, it is generally assumed that a cellulose molecule is a chain of anhydroglucose or cellobiose units bound by oxygen bridges. These long chains of anhydroglucose with oxygen bridges are very stable and have good flexibility. These properties are utilized in the estrogen agonist-containing patch according to the present invention to render the pressure-sensitive adhesive sufficiently cohesive, this is required to remove the patch from the skin without leaving any residue after completed application. The pressure-sensitive adhesive comprises ethylcellulose in a proportion of 5-25% wt., preferably 8-14% wt.

Examples of esters of colophony include, for example, methyl ester, glycerol ester, pentaerythritol ester, pentaerythritol ester modified with maleic acid, glycerol ester modified with maleic acid, and triethylene glycol ester. The proportion of colophony esters in the estrogen agonist-containing pressure-sensitive adhesive amounts to 50-90% wt., preferably 60-80% wt.

The pressure-sensitive adhesive may comprise esters of hydrogenated colophony either alone or together with esters of non-hydrogenated colophony.

Particularly preferred esters of colophony include triethylene glycol ester, glycerol ester, and pentaerythritol ester of hydrogenated colophony.

Lauric acid is a basic carboxylic acid having 12 C-atoms. It increases the penetration of estradiol through the skin. The proportion of lauric acid contained in the pressure-sensitive adhesive amounts to 1-20% wt., preferably 2-15% wt.

The reservoir of the recrystallization-free, estrogen agonist-antagonist-containing patch with sufficient active substance release comprises estradiol and its pharmaceutically acceptable derivatives alone or in combination with gestagens at a total concentration of 1 varies as 15% wt., relative to all of the reservoir components, namely in a molar ratio of 1:1 to 1:10.

The estrogen agonist-antagonist-containing reservoir may comprise at least one component of the group including anti-ageing agents, plasticizers, antioxidants, and absorption improvers. Suitable plasticizers are known to those skilled in the art and are described, for example, in DE 37 43 946. Usually, the proportion of plasticizers in the estrogen agonist-antagonist-containing reservior amounts to up to 5%-wt.

In addition, the active substance-containing reservoir also comprises anti-ageing agents in a concentration of up to 1% wt. These are known to the skilled artisan and are described, for example, in DE 37 43 946.

The materials for the impermeable backing layer and the removable protective layer are also known to the skilled artisan (e.g., DE 38 43 239).

The estrogen agonist-antagonist-contaihing reservoir may either be produced from solution or from the melt.

Moreover, the reservoir may consist of several layers.

In case the reservoir has an insufficient self-tackiness to the skin, it may be provided with an additional pressure-sensitive adhesive layer which pressure-sensitive adhesive edge. This ensures that the transfermal patch adheres to the skin over the whole application period.

A particularly preferred structure of the transdermal estrogen agonist-containing patch is a matrix system wherein, as is generally known, the matrix controls the active substance release and complies with the square root-law according to Higuchi. However, it is understood that a membrane system may well be of advantage in particular cases. In this case, a membrane controlling the active substance release is located between the reservoir and the pressure-sensitive adhesive layer.

The thickness of the transdermal patch depends on the therapeutic requirements and may be adapted accordingly. Usually, it ranges from 0.03-0.6 mm.

The transdermal patches of this invention are applied directly to the skin of the patient. Preferred application sites are clean, dry and intact areas of skin, below the waistline on the trunk of the body. Preferably the buttock, hip or abdomen area is chosen since these areas of skin wrinkle least during body movement.

In the following examples, specific embodiments of the present invention are set forth. All parts and percentages are by weight, unless indicated otherwise.

### Example 1

A seven day patch is prepared by dissolving (-)-cis-6-phenyl-5-[4-(2-pyrrolidin-1-yl-ethoxy)-phenyl]-5,6,7,8-tetrahydronaphthalene-2-ol in a polymer matrix to form a patch which delivers about 10-500 µg per day for seven days.

### Example 2

A fourteen day transdermal patch is prepared by dissolving (-)-cis-6-phenyl-5-[4-(2-pyrrolidin-1-yl-ethoxy)-phenyl]-5,6,7,8-tetrahydronaphthalene-2-ol in a polymer matrix to form a patch which delivers about 10-500 µg per day for fourteen days.

## Claims

1. The use of an effective amount of an estrogen agonist-antagonist having the following formula
wherein G is 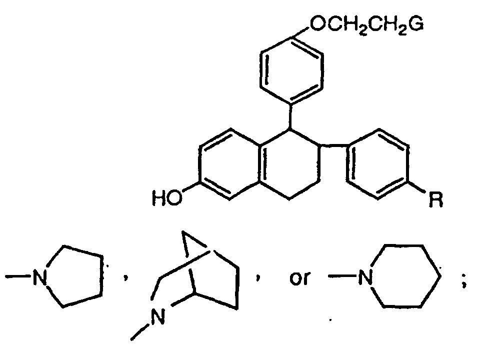 and
R is H, OH, F or Cl.
for the preparation of a medicament for the treatment by transdermal means of a human in need of estrogen agonist-antagonist therapy.

2. The use as claimed in Claim 1 wherein said transdermal means is a transdermal patch.

3. The use as claimed in Claim 1 wherein said transdermal means is a hydrogel composition.

4. The use as claimed in Claim 2 wherein said transdermal patch is a drug reservoir patch.

5. A transdermal patch comprising an estrogen agonist-antagonist according to the formula of Claim 1.

6. A transdermal patch as claimed in Claim 5 wherein said estrogen agonist-antagonist is administered to a human over a period of 3 to 14 days in substantially equal amounts of 10-500 micrograms per day.

7. A transdermal patch comprising a matrix reservoir and a pharmaceutically effective amount of (-)-cis-6-phenyl-5-[4-(2-pyrrolidin-1-yl-ethoxy)-phenyl]-5,6,7,8-tetrahydronaphthalene-2-ol or a pharmaceutically acceptable salt.

## Patentansprüche

1. Verwendung einer wirksamen Menge eines Östrogenagonist-Antagonisten folgender Formel wobei G für 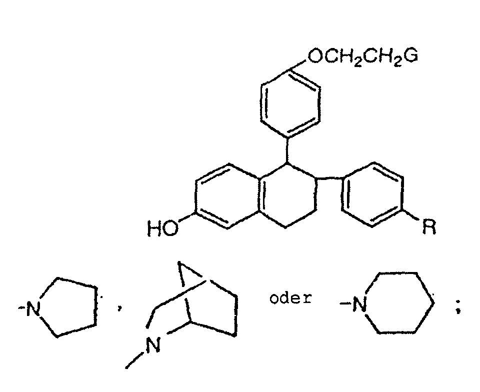 steht und R für H, OH, F oder Cl steht,
für die Zubereitung eines Medikaments für die Behandlung durch transdermale Mittel bei einem Menschen, der eine Östrogenagonist.Antagonistentherapie benötigt.

2. Verwendung nach Anspruch 1, wobei es sich bei dem transdermalen Mittel um ein transdermales Pflaster handelt.

3. Verwendung nach Anspruch 1, wobei es sich bei dem transdermalen Pflaster um eine Hydrogelzusammensetzung handelt.

4. Verwendung nach Anspruch 2, wobei es sich bei dem transdermalen Pflaster um eine Arzneimittel-Reservoirpflaster handelt.

5. Transdermales Pflaster umfassend einen der Formel von Anspruch 1 entsprechenden Östrogenagonist-Antagonisten handelt.

6. Transdermales Pflaster nach Anspruch 5, wobei der Östrogenagonist-Antagonist einem Menschen über eine Zeitspanne von 3 bis 14 Tagen in im Wesentlichen gleichen Mengen von 10 - 500 Mikrogramm pro Tag verabreicht wird.

7. Transdermales Pflaster umfassend ein Matrixreservoir und eine pharmazeutisch wirksame Menge (-)-cis-6-Phenyl-5-[4-(2-pyrrolidin-1-yl-ethoxy)-phenyl]-5,6,7,8-tetrahydronaphthalin-2-ol oder eines pharmazeutisch akzeptablen Salzes desselben.

## Revendications

1. Utilisation d'une quantité efficace d'un agent agoniste-antagoniste d'oestrogènes ayant la formule où G est 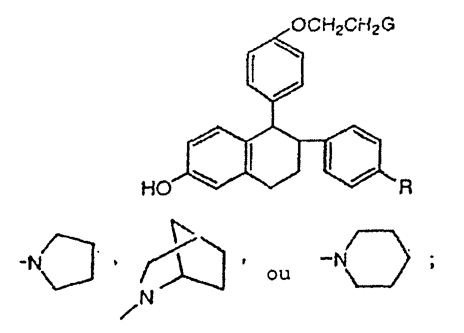 et R est H, OH, F ou Cl,
en vue de la préparation d'un médicament pour le traitement, par des moyens transdermiques, d'un homme ou d'une femme, ayant besoin d'une thérapie agoniste-antagoniste d'oestrogènes.

2. Utilisation selon la revendication 1, dans laquelle ledit moyen transdermique est un timbre transdermique.

3. Utilisation selon la revendication 1, dans laquelle ledit moyen transdermique est une composition d'hydrogel.

4. Utilisation selon la revendication 2, dans laquelle ledit timbre transdermique est un timbre à réservoir de médicament.

5. Timbre transdermique comprenant un agent agoniste-antagoniste d'oestrogènes selon la formule de la revendication 1.

6. Timbre transdermique selon la revendication 5, dans lequel ledit agent agoniste-antagoniste d'oestrogènes est administré à un homme ou à un femme sur une période allant de 3 à 14 jours dans des quantités substantiellement égales de 10-500 micro grammes par jour.

7. Timbre transdermique comprenant un réservoir à matrice et une quantité, efficace du point de vue pharmaceutique, du (-)-cis-6-phényl-5-[4-(2-pyrrolidin-1-yl-éthoxy)-phényl]-5,6,7,8-tétrahydronaphtalène-2-ol ou d'un sel acceptable du point de vue pharmaceutique.
